# EUROPÄISCHE PATENTSCHRIFT

(11) **EP 1 408 832 B1**
(45) Veröffentlichungstag und Bekanntmachung des Hinweises auf die Patenterteilung: **15.03.2006**
(21) Anmeldenummer: 02730150.6
(22) Anmeldetag: 12.04.2002
(51) Int. Cl.: A61B 5/087

(54) **STEUERUNGSEINRICHTUNG ZUR VORGABE EINES ATEMGASDRUCKES**
CONTROL DEVICE FOR SETTING A BREATHING GAS PRESSURE
DISPOSITIF DE COMMANDE POUR DEFINIR UNE PRESSION DE GAZ RESPIRATOIRE

(30) Priorität: 12.04.2001 DE 10118475
(43) Veröffentlichungstag der Anmeldung: 21.04.2004
(73) Patentinhaber: MAP Medizin-Technologie GmbH, 82152 Martinsried (DE)
(72) Erfinder: MEIER, Jörg, 81547 München (DE); MADAUS, Stefan, 82152 Krailling (DE); HEIDMANN, Dieter, 82541 Münsing (DE); SCHÄTZL, Stefan, 82362 Weilheim (DE); BRANDMEIER, Richard, 86492 Egling (DE)
(74) Vertreter: Heunemann, Dieter
(86) Internationale Anmeldenummer: PCT/EP2002/004095
(87) Internationale Veröffentlichungsnummer: WO 2002/082997

(56) Entgegenhaltungen:
- WO-A-93/09834
- WO-A-95/32016
- US-A- 5 458 137
- TESCHLER H ET AL: "INTELLIGENT CPAP SYSTEMS: CLINICAL EXPERIENCE" THORAX, LONDON, GB, Bd. 53, Nr. SUPPL 3, Oktober 1998 (1998-10), Seiten S49-S54, XP000879277

## Beschreibung

### Gebiet der Erfindung

Die Erfindung betrifft eine Steuerungseinrichtung zur Vorgabe eines Atemgasdruckes im Rahmen der Diagnose und/oder Therapie schlafbezogener Atmungsstörungen gemäß dem Oberbegriff von Patentanspruch 1.

Insbesondere betrifft die Erfindung auch ein Schlaftherapiegerät mit selbstabstimmender Druckregelung wobei insbesondere der Atemgasdruck während eines Inspirationsvorganges, der Atemgasdruck während eines Expirationsvorganges und der zeitliche Verlauf der Druckänderungen in Abhängigkeit von dem physiologischen Zustand eines Patienten abgestimmt werden.

### Hintergrund der Erfindung

Aus WO 95/32016 ist bekannt, schlafbezogene Atmungsstörungen, insbesondere in Verbindung mit Obstruktionen im Bereich der oberen Atemwege durch eine überdruckbeatmung mit einem ggf. alternierenden, jedoch überwiegend über dem Umgebungsdruckniveau liegenden Atemgasdruck zu behandeln, wobei die Einstellung des Atemgasdruckes unter Auswertung des zeitlichen Verlaufs des Atemgasflusses erfolgt.

Durch eine allgemein als CPAP-Therapie bezeichnete Überdruckbeatmung wird es möglich, im Bereich der oberen Atemwege eine "pneumatische Schienung" herbeizuführen. Durch diese pneumatische Schienung wird etwaigen Obstruktionen im Bereich der oberen Atemwege vorgebeugt.

Um eine möglichst hohe physiologische Verträglichkeit der CPAP-Therapie zu gewährleisten, ist man bestrebt, den Beatmungsdruck möglichst gering zu halten und insbesondere während einer Expirationsphase gegenüber der Inspirationsphase abzusenken.

Günstige Beatmungsdruckpegel können durch den Patienten selbst gewählt werden oder beispielsweise auch unter ärztlicher Betreuung im Rahmen des Aufenthalts des Patienten in einem Schlaflabor ermittelt werden. Untersuchungen haben jedoch gezeigt, dass sowohl die vom Patienten selbst gewünschten, als auch die im Rahmen des Aufenthalts in einem Schlaflabor ermittelten Therapiedrücke für den Inspirationsdruck sowie für den üblicherweise etwas geringeren Expirationsdruck teilweise deutlich über den für den überwiegenden Teil der Schlafphase, insbesondere zur Erreichung des Schienungseffektes erforderlichen Inspirations- und Expirationsdruckpegeln liegen.

### Aufgabe der Erfindung

Unter dem Eindruck dieses Problems liegt der Erfindung die Aufgabe zugrunde, eine Steuerungseinrichtung zur Bestimmung eines Atemgasdruckes im Rahmen der Diagnose und/oder Therapie schlafbezogener Atmungsstörungen zu schaffen, durch welche ein insbesondere auf den momentanen physiologischen Zustand des Patienten verbessert abgestimmter Atemgasdruck gewährleistet ist, ohne dass im Zuge der Optimierung des Atemgasdruckes der Schlafverlauf des Patienten erheblich beeinträchtigt wird.

### Erfindungsgemäße Lösung

Diese Aufgabe wird erfindungsgemäß gelöst durch eine Steuerungseinrichtung gemäß Patentanspruch 1.

Dadurch wird es auf vorteilhafte Weise möglich, die für den momentanen physiologischen Zustand des Patienten günstigste Atemgasdruckcharakteristik zu ermitteln, ohne dass der Patient im Zusammenhang mit der Optimierung des Atemgasdruckes erheblichen Störeinflüssen ausgesetzt ist.

Gem. einer besonders bevorzugten Ausführungsform der Erfindung wird das Spezifikationssignal zur Umschaltung zwischen den wenigstens zwei Druckermittlungsmodi in Abhängigkeit von mehreren, zusammenfassend betrachteten Bewertungssignalen oder Bewertungssignal-Datensätzen generiert. Die Bewertungssignale beschreiben die Erfüllung vorgegebener, auf den Atemgasfluss angewandter Kriterien. Diese vorgegebenen Kriterien werden gemäß einer besonders bevorzugten Ausführungsform der Erfindung zumindest teilweise adaptiv variiert. Dadurch wird es möglich, die zur Generierung der Bewertungssignale und damit zur Einstellung des Atemgasdruckes maßgeblichen Regelungsparameter adaptiv abzustimmen.

Gem. einer besonders bevorzugten Ausführungsform der Erfindung wird wenigstens eines der Bewertungssignale durch eine Apnoe-Detektionseinrichtung generiert.

Gem. einer besonders bevorzugten Ausführungsform der Erfindung wird vorzugsweise ein weiteres Bewertungssignal durch eine Hypopnoe-Detektionseinrichtung generiert.

Gem. einer besonders bevorzugten Ausführungsform der Erfindung ist weiterhin eine Krümmungs-Beurteilungseinrichtung vorgesehen, durch welche auf Grundlage von Atemflusssignalen ebenfalls ein Bewertungssignal generiert wird.

Ebenfalls vorzugsweise in Kombination mit den vorangehend beschriebenen Maßnahmen wird ein hinsichtlich eines Normalatmungszustandes indikatives Bewertungssignal mittels eines Normalatmungsdetektors generiert.

Gem. einer besonders bevorzugten Ausführungsform der Erfindung ist weiterhin eine Atemstillstands-Detektionseinrichtung vorgesehen, die ebenfalls ein Bewertungssignal generiert.

Insbesondere in Kombination mit den vorangehend genannten Detektionseinrichtungen ist weiterhin eine Leckage-Detektionseinrichtung vorgesehen zur Erfassung eines Leckagezustandes, wobei im Falle erkannter Leckage die Druckregelung mittels einer Leckage-Druckregelung erfolgt, die in weiten Zügen unabhängig von den ersten und zweiten Druckregelungsmodi arbeitet.

Die Generierung der Bewertungssignale durch die vorangehend genannten Detektionseinrichtungen erfolgt vorzugsweise auf Grundlage von Merkmalen, die aus hinsichtlich des Atemgasflusses indikativen Signalen oder für Eigenschaften des Atemgasflusses indikativen Signalen extrahiert werden. Die Leckage-Detektionseinrichtung beobachtet vorzugsweise den Atemgasfluss und/oder den Atemgasdruck über einen vorgegebenen Beobachtungszeitraum der sich vorzugsweise mitlaufend über die zurückliegenden 60 Sekunden erstreckt. Das hierbei generierte Signal wird vorzugsweise einer Leckage-Druckregeleinrichtung zugeführt, die eine Umschaltung in einen Leckage-Modus veranlasst wenn das Ausgangssignal der Leckage-Detektionseinrichtung innerhalb eines ebenfalls mitlaufenden Zeitfensters von z.B. 240 bis 360 Sekunden präsent ist.

So erfolgt vorzugsweise die Generierung mehrerer Bewertungssignale durch mehrere Vorauswertungsmodule, welche Ergebnisse bereits vorangehend, durchgeführter Auswertungsprozeduren liefern. So kann beispielsweise die Länge eines Atemzuges, die zeitliche Länge eines Ausatemvorganges, die zeitliche Länge eines Einatemvorganges und beispielsweise auch das aus dem zeitlichen Verlauf des Atemgasstromes errechnete Atemzugsvolumen modular ermittelt werden, wobei die hierbei gewonnenen Ergebnisse den Bewertungssignal-Generierungsmodulen zur Verfügung stehen oder von diesen ausgelesen werden.

Weiterhin wird gem. einer besonders bevorzugten Ausführungsform der Erfindung von wenigstens einem der Vorauswertungsmodule eine Rückwärtskorrelation des momentanen Verlaufes des Atemgasstromes mit einem Referenzatemgasstrom oder einem, aus zurückliegenden Atemzyklen generierten Atemzugsverlauf betrachtet.

Gem. einer besonders bevorzugten Ausführungsform werden innerhalb eines Vorauswertungsmodules, beispielsweise Krümmungseigenschaften des zeitlichen Verlaufes des Atemgasstromes, berücksichtigt.

Gem. einer besonders bevorzugten Ausführungsform der Erfindung ist ein Vorauswertungsmodul vorgesehen, das eine Atemzugslängenerfassungseinrichtung bildet zur Generierung eines hinsichtlich der zeitlichen Länge eines Atemzyklus indikativen Atemzyklus-Zeitdauersignales. Dieses Signal steht mehreren Bewertungssignal-Generierungsmodulen zur Verfügung.

In weiterhin vorteilhafter Weise befindet sich unter den Vorauswertungsmodulen auch eine Expirations-Zeitdauererfassungseinrichtung zur Bereithaltung eines hinsichtlich der zeitlichen Länge einer Expirationsphase indikativen Signales, welches wiederum vorzugsweise mehreren Bewertungssignal-Generierungssystemen zur Verfügung stehen kann.

Vorzugsweise ist weiterhin unter den Vorauswertungsmodulen auch eine Inspirations-Zeitdauererfassungseinrichtung vorgesehen zur Bereithaltung eines hinsichtlich der zeitlichen Länge einer Inspirationsphase indikativen Signales. In vorteilhafter Weise erfolgt eine Analyse des Atemgasflusssignales auf Grundlage einer Zeitreihenanalyse.

In weiterhin vorteilhafter Weise ist unter den Vorauswertungsmodulen eine Volumenerfassungseinrichtung vorgesehen zur Bereithaltung eines hinsichtlich eines Atemzugvolumens indikativen Signales.

Zur Bereithaltung eines hinsichtlich einer Rückwärtskorrelationsbeziehung des momentanen Atemzuges mit einem hinsichtlich zurückliegender Atemzüge repräsentativen Atemzugsverlauf indikativen Signal ist gem. einer besonders bevorzugten Ausführungsform der Erfindung in Form eines Vorauswertungsmodules eine Korrelationserfassungseinrichtung vorgesehen. Die Korrelation des aktuellen Atemzugsverlaufes mit dem Referenzverlauf kann nach Maßgabe mehrerer ggf. adaptiv optimierter Korrelationskriterien beurteilt und vorzugsweise skalar werden.

Die hinsichtlich der Korrelationseigenschaften bereitgehaltenen Auswertungen können vorzugsweise mehreren Bewertungssignal-Generierungsmodulen zur Verfügung gestellt werden.

Gem. einer besonders bevorzugten Ausführungsform der Erfindung umfaßt das System weiterhin eine Krümmungsermittlungseinrichtung zur Ermittlung von hinsichtlich der Krümmung des Atemgasstromverlaufes indikativen Signalen. Diese hinsichtlich der Krümmung, d.h. zweiten der Ableitung des Atemgasstromverlaufes indikativen Signale können beispielsweise im Zusammenhang mit Schwellwertvergleichen bei der Generierung der Bewertungssignale berücksichtigt werden.

Gem. einer besonders bevorzugten Ausführungsform der Erfindung generiert die Apnoe-Detektionseinrichtung das Bewertungssignal auf Grundlage des seitens der Expirations-Zeitdauererfassungsvorrichtung erzeugten Zeitsignales.

Die Hypopnoe-Detektionseinrichtung generiert das Bewertungssignal vorzugsweise auf Grundlage des durch die Volumenerfassungseinrichtung determinierten Atemzugvolumens und eventuell durch die Korrelationserfassungseinrichtung determinierten Rückwärtskorrelationsbeziehung.

Die Normalatmungs-Detektionseinrichtung generiert das Bewertungssignal vorzugsweise auf Grundlage des Auswertungsergebnisses der Rückwärtskorrelationsbeziehung.

Die Atemstillstands-Detektionseinrichtung generiert das Bewertungssignal vorzugsweise auf Grundlage des Atemgas-Flusssignales.

Gem. einer besonders bevorzugten Ausführungsform der Erfindung ist weiterhin eine Schnarcherfassungseinrichtung vorgesehen zur Generierung eines hinsichtlich eines Schnarchereignisses indikativen und vorzugsweise auch dieses Schnarchereignis klassifizierenden Signales. Dieses, das Schnarchereignis klassifizierende Signal wird vorzugsweise für mehrere Bewertungssignal-Generierungsmodule bereitgehalten.

Die Schnarcherkennungseinrichtung generiert das hinsichtlich des Schnarchereignisses indikative Signal vorzugsweise auf Grundlage einer Null-Durchgangsbetrachtung der ersten Ableitung eines hinsichtlich des Atemgasdruckes repräsentativen Signales.

Die Entscheidung zur Durchführung einer Umschaltung zwischen den wenigstens zwei unterschiedlichen Drucksteuerungsmodi erfolgt vorzugsweise auf Grundlage einer Auswertung mehrerer Bewertungssignale. Bei der Auswertung dieser Bewertungssignale können Gewichtungsfaktoren berücksichtigt werden, durch welche die nach den unterschiedlichsten Kriterien vorzugsweise modular generierten Bewertungssignale berücksichtigt werden.

Diese Gewichtungsfaktoren können auf Grundlage von Kriterien festgelegt werden, die sich aus dem Atemgasstrom und/oder dem Druck generieren. Beispielsweise wird der Gewichtungsfaktor aus dem zurückliegenden Verlauf des Atemgasstromes gewonnen. Die Gewichtungsfaktoren werden vorzugsweise in Abstimmung zueinander festgelegt. Die zeitliche Änderung dieser Gewichtungsfaktoren wird vorzugsweise durch Dämpfungskriterien bzw. Schwellwerte auf ein vorbestimmtes Maß gedämpft bzw. gesetzt.

Gem. einer besonders bevorzugten Ausführungsform der Erfindung unterscheiden sich die beiden Druckanpassungsmodi insbesondere im Hinblick auf deren Reaktionsverhalten auf bestimmte, aus dem zeitlichen Verlauf des Atemgasstromes extrahierte Merkmale. So ist es beispielsweise möglich, den ersten Druckermittlungsmodus derart abzustimmen, dass eine Druckänderung in Abhängigkeit von einem Schwellwertkriterium erfolgt, wobei der entsprechende Schwellwert auf einem höheren Schwellwertpegel liegt, als ein vergleichbarer Schwellwert in dem zweiten Drukkermittlungsmodus.

### Kurzbeschreibung der Figuren

Weitere Einzelheiten der Erfindung ergeben sich aus der nachfolgenden Beschreibung, wobei auch auf die beigefügten Zeichnungen Bezug genommen werden wird. Die beigefügten Zeichnungen zeigen:
- Fig. 1: eine Schemaansicht zur Erläuterung des erfindungsgemäßen Druckregelungskonzeptes zur Einstellung des Atemgasdruckes;
- Fig. 2: den zeitlichen Verlauf des Atemgasstromes über insgesamt fünf Atemzyklen hinweg;
- Fig. 3: ein Flussdiagramm zur Erläuterung eines bevorzugten Verfahrensablaufes zur Auffindung des Beginns einer Inspirationsphase;
- Fig. 4: eine Konzeptdarstellung zur Erläuterung der zur Erkennung eines Apnoe-Ereignisses relevanten Zeitfenster;
- Fig. 5: eine Skizze zur Erläuterung von Änderungen hinsichtlich des Atemzugvolumens;
- Fig. 6: eine Konzeptdarstellung zur Erläuterung der im Zusammenhang mit einer Hypopnoe-Erfassung relevanten Zeitfenster;
- Fig. 7: eine Konzeptdarstellung zur Erläuterung der Rückwärtskorrelationsbetrachtung, wie sie beispielsweise Eingang in die seitens des Normalatmungsdetektors und des Krümmungsdetektors generierten Bewertungssignale Eingang findet;
- Fig. 8: eine Konzeptdarstellung zur Erläuterung einer bevorzugten Variante des zur Erkennung einer Flusslimitation eingesetzten Krümmungsdetektors, der sowohl die zweite Ableitung des Atemgasstromes als auch die Ergebnisse der Rückwärtskorrelation (Fig. 7) erarbeitet;
- Fig. 9: eine Konzeptdarstellung zur Erläuterung der Funktionsweise der Schnarcherkennungseinrichtung;
- Fig. 10: eine Konzeptdarstellung zur Erläuterung des Atemstillstand-Detektors;
- Fig.: 12 eine Konzeptdarstellung zur Erläuterung des Normal-Atemdetektors;
- Fig. 13: eine Konzeptdarstellung zur Erläuterung des Leckagedetektors.

### Ausführliche Beschreibung unter Bezugnahme auf die Zeichnung

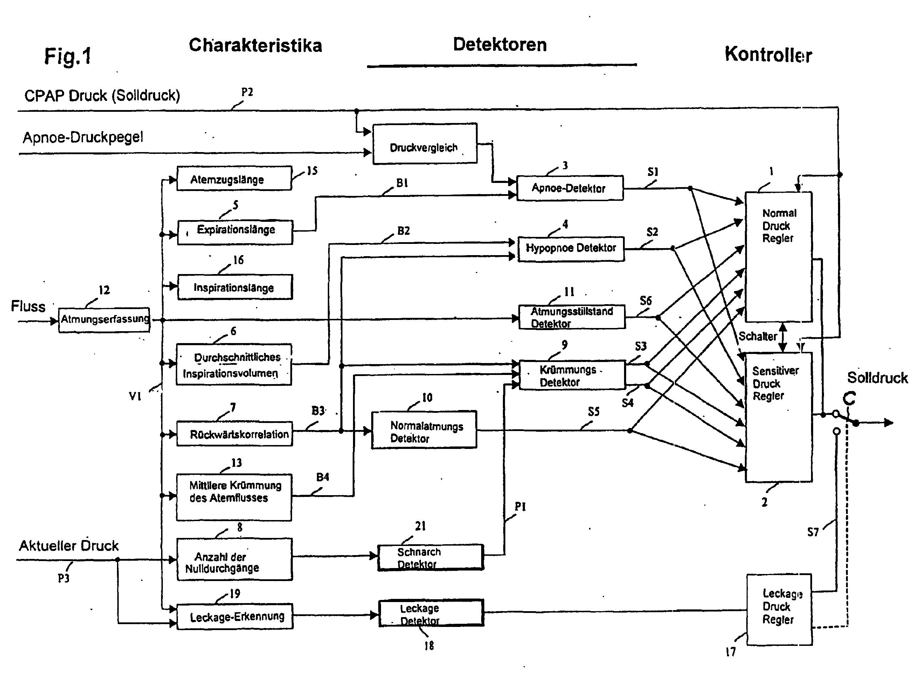

Das in Figur 1 dargestellte regelungstechnische System wird vorzugsweise durch einen elektronischen Schaltkreis unter Verwendung speicherprogrammierbarer Komponenten realisiert.

Das in Figur 1 dargestellte Drucksteuerungssystem umfasst einen Primärbetriebsdruckregler 1 zur Durchführung einer Atemgasdruckanpassung nach Maßgabe eines ersten Druckregelungsmodus.

Parallel zu diesem Regelbetriebsdruckregler 1 ist ein Sekundärbetriebsdruckregler 2 vorgesehen, durch welchen eine Anpassung des Atemgasdruckes nach Maßgabe eines zweiten Druckregelungsmodus erfolgt, der sich in Einzelheiten von dem Druckregelungsmodus des Regelbetriebdruckreglers 1 unterscheidet.

Die Entscheidung , welcher der wenigstens zwei unterschiedlichen Druckregler 1, 2 zum Einsatz kommt, wird in Abhängigkeit von einem Selektionssignal getroffen, das aus einer zusammengefassten Auswertung mehrerer Bewertungssignalen S1 ... S6 generiert wird.

Die zusammengefasste Auswertung der Bewertungssignale S1..... S6 erfolgt bei dem dargestellten Ausführungsbeispiel bereits im Bereich der Druckregler 1, 2. Hierzu stehen diese Bewertungssignale S1.... S6 jedem der Druckregler 1, 2 zur Verfügung.

In Abhängigkeit von diesen Bewertungssignalen kann bestimmt werden, ob der Beatmungsdruck nach Maßgabe des Primärbetriebsdruckreglers oder nach Maßgabe des Sekundärbetriebsdruckreglers 2 erfolgt.

Das Bewertungssignal S1 wird durch einen Apnoe-Detektor 3 generiert und liegt sowohl an dem Primärbetriebsdruckregler 1, als auch an dem Sekundärbetriebsdruckregler 2 an. Der Apnoe-Detektor 3 generiert das Bewertungssignal S1 auf Grundlage eines hinsichtlich der Expirationsdauer repräsentativen Signales B1. Dieses hinsichtlich der Expirationsdauer repräsentative Signal B1 wird durch eine Vorauswertung bereitgehalten die hier durch ein Expirationsdauermodul durchgeführt wird.

Das Bewertungssignal S2 wird durch den Hypopnoe-Detektor 4 generiert und steht ebenfalls beiden Druckreglern 1, 2 zur Verfügung. Der Hypopnoe-Detektor 4 generiert das Bewertungssignal S2 auf Grundlage eines hinsichtlich des mittleren Atemzugvolumens indikativen Signales B2 sowie auf Grundlage von Informationen/Signalen B3 hinsichtlich einer Rückwärtskorrelationsbeziehung. Diese hinsichtlich des mittleren Atemzugvolumens indikativen Signale B2 werden durch ein Atemzugvolumenmodul 6 bereitgestellt. Die hinsichtlich der Rückwärtskorrelationsbeziehung indikativen Informationen/Signale B3 werden durch ein Back-Correlation-Modul 7 bereitgestellt. Die seitens des Back-Correlation-Modul 7 als Signale B3 bereitgehaltenen Auswertungen stehen gemeinsam mit hinsichtlich des Krümmungsverlaufes der Atemgasflusskurve indikativen und seitens eines Krümmungsbestimmungs-Modules 13 generierten, Signalen B4 einer Krümmungsauswertungseinheit 9 zur Verfügung. Die Krümmungsauswertungseinheit 9 erzeugt unter Rückgriffnahme auf die Signale/Informationen B3, B4 die Bewertungssignale S3 und S4. Die Generierung des Bewertungssignales S4 erfolgt unter Berücksichtigung der hinsichtlich eines Schnarchereignisses indikativen Signale P1.

Ebenfalls unter Rückgriffnahme auf die Auswertungen des Back-Correlation-Modules 7 erfolgt die Generierung eines, eine normale Atmung angebenden Bewertungssignales S5 durch einen Normalatmungs-Detektor 10.

Ein etwaiger Atemstillstand wird durch ein Bewertungssignal S6 beschrieben. Das Bewertungssignal S6 wird durch einen Atemstillstandsdetektor 11 generiert, der unmittelbar Zugriff auf die hinsichtlich des zeitlichen Verlaufes des Atemgasstromes indikativen und seitens eines Atemgasflussdetektors 12 generierten Messsignale V1 hat.

Durch das erfindungsgemäße System werden durch Analyse des Atemflusssignales V1 vorzugsweise noch weitere Auswertungsergebnisse bereitgehalten wie beispielsweise Zeitangangaben zur Gesamtdauer eines Atemzuges (Atemphasen-Zeitmesseinrichtung 15) oder auch zur Dauer der Inspirationsphase (Inspirationszeit-Messeinrichtung 16).

Bei dem dargestellten System ist eine Signal-Bypasseinrichtung vorgesehen, über welche bei erkannter Leckage beispielsweise aufgrund einer unzureichenden Maskendichtwirkung, die Steuerung des Atemgasdruckes nach einem speziell auf diesen Störzustand abgestimmten Steuerungskonzept erfolgt. Hierzu ist ein Leckage-Druckregler 17. Die Ansteuerung dieses Leckage-Druckreglers 17 erfolgt über eine Leckage-Sensoreinrichtung 18, 19, welche die hinsichtlich einer unzulässig hohen Systemleckage indikativen Informationen aus dem Atemgasflusssignal V1 und vorzugsweise auch aus den Atemgasdrucksignalen P2 und P3 extrahiert.

Im Falle einer durch die Leckage-Sensoreinrichtung 18, 19 erkannten, unzulässigen Systemleckage erfolgt ein Wechsel aus dem Primär- oder Sekundärdruckregelungsmodus in einen Leckage-Druckregelungsmodus.

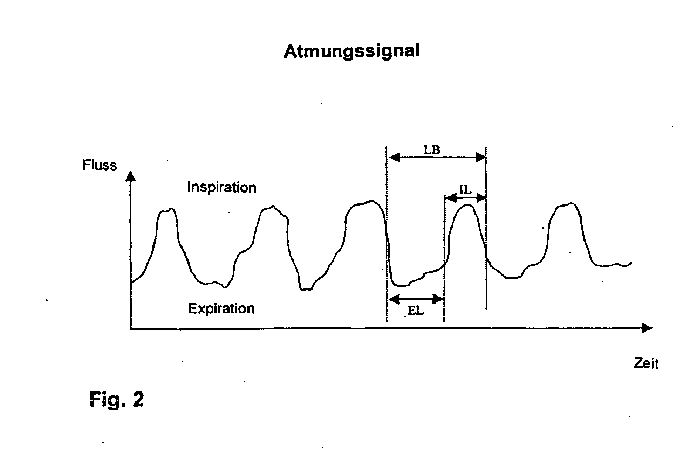

In Figur 2 zeigt einen typischen Verlauf des Atemgasflusses dargestellt. Das Intervall LB kennzeichnet einen vollen Atemzyklus. Das Intervall EL entspricht der Expirationsphase, das Intervall IL der Inspirationsphase.

Aus dem gesamten Atmungszyklus kann der mittlere Atemgasflusses errechnet werden. Die Expirationsphase ist insbesondere für die Rückwärtskorrelationsbetrachtung relevant.

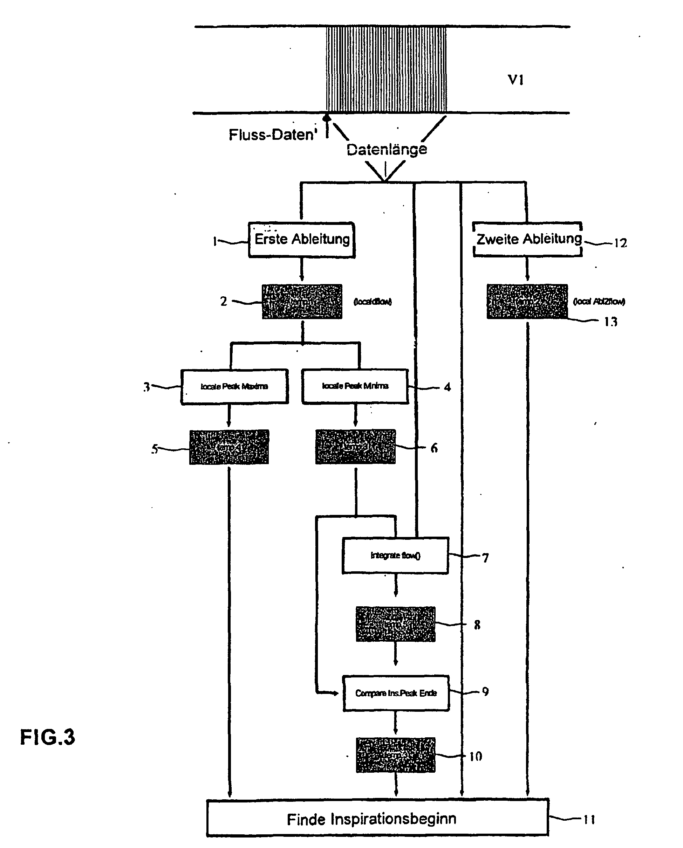

Das Ende bzw. der Beginn der Atemphase sowie deren Inspirations- und Exspirationsintervalle erfolgt vorzugsweise nach Maßgabe eines Untersuchungsablaufes wie, er in Form eines Flussdiagramms in Figur 3 angegeben ist. Hierbei werden aus dem Flussignal V1 die erste Ableitung des Atemgasflusses berechnet, um die Steigung zu erhalten, und werden in ein Datenfeld bzw. in mehrere Variablen (Figur 3; Bezugszeichen 2) gespeichert. Die Steigungspunkte werden anschließend nach ihrer negativen und positiven Steigung (Figur 3; Bezugszeichen 3, 4) sortiert und in Datenfelder bzw. in mehreren Variablen (Figur 3; Bezugszeichen 5, 6) gespeichert. Der negative Steigungspunkte entspricht dem Inspirationsende des Atemzugs. Um das Inspirationsende eines Atemzugs von Störungen zu detektieren wird links vom Steigungspunkt (zeitlich davor) eine bestimmte Anzahl des Flussignal V1 beispielsweise 80 Datenpunkte integriert (Figur 3; Bezugszeichen 7). Das ermittelte Volumen wird in einem Datenfeld bzw. in mehrere Variablen (Figur 3; Bezugszeichen 8) gespeichert. Anschließend werden die negativen Steigungspunkte in der Inspirationsendeerkennung 9 ermittelt. Die Kriterien für die Detektierung des Inspirationsende ist das Volumen und ein minimaler Zeitabstand vom letzten Inspirationsende beispielsweise 2 Sekunden. Das Inspirationsende wird anschließend in einem Datenfeld bzw. in mehreren Variablen (Figur 3; Bezugszeichen 10) gespeichert.

Für die eindeutige Erkennung des Inspirationsstarts werden mehrere Berechnungen benötigt. Aus der ersten Ableitung des Flussignals V1 wird hinreichend das Enden der Exspiration bzw. der Beginn der Inspiration erkannt. Für eine genaue Bestimmung der Inspirationsstarts wird die zweite Ableitung des Flussignals V1 berechnet. Aus der zweiten Ableitung werden die Wendepunkte ermittelt (Figur 3; Bezugszeichen 12) und in ein Datenfeld bzw. in mehrere Variablen (Figur 3; Bezugszeichen 13) gespeichert. Aus der ersten und zweiten Ableitung wird der Start der Inspirationsphase mit Hilfe des aktuellen Flussignals V1 ermittelt.

### Die Detektoren

### Apnea-Detektor

Der in Figur 1 durch das Bezugszeichen 3 gekennzeichnete Apnea-Detektor 3 kann das Bewertungssignal S1 auf Grundlage des hinsichtlich der Expirationsdauer indikativen Signales B1 erzeugen.

Gemäß einem bevorzugten Funktionsprinzip des Apnea-Detektors wird zunächst überprüft, ob die als Expirationszeit erfaßte Zeitdauer (Figur 2 EL) einen vorgegebenen Schwellenwert von beispielsweise 10 Sekunden übersteigt. Ein derartiger Zustand wird als Ereignis angesehen.

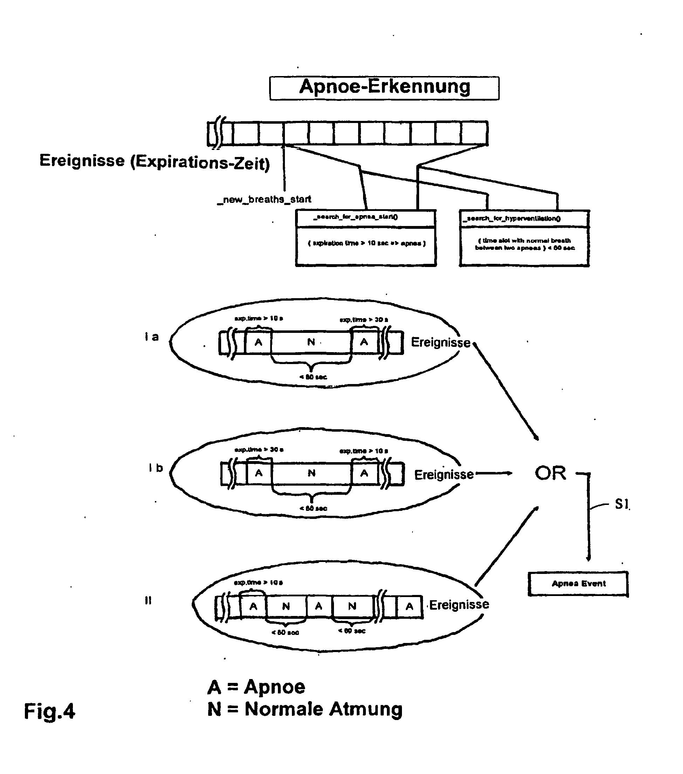

Wie in Figur 4 schematisch dargestellt ist, wird das einen Apnoe-Zustand anzeigende Bewertungssignal S1 generiert, wenn entweder innerhalb eines vorgegebenen Zeitfensters von beispielsweise 80 Sekunden zwei aufeinanderfolgende Atemstillstandsphasen mit einer Zeitdauer von wenigstens 30 Sekunden - oder drei folgenden Stillstandsphasen mit einer Zeitdauer von wenigstens 10 Sekunden erfaßt werden - oder auf ein Ereignis mit einer Expirationsdauer von mehr als 10 Sekunden innerhalb der nächsten 60 Sekunden ein weiteres Ereignis mit mehr als 30 Sekunden Exspirationszeitdauer folgt.

### Atemstillstandsdetektor

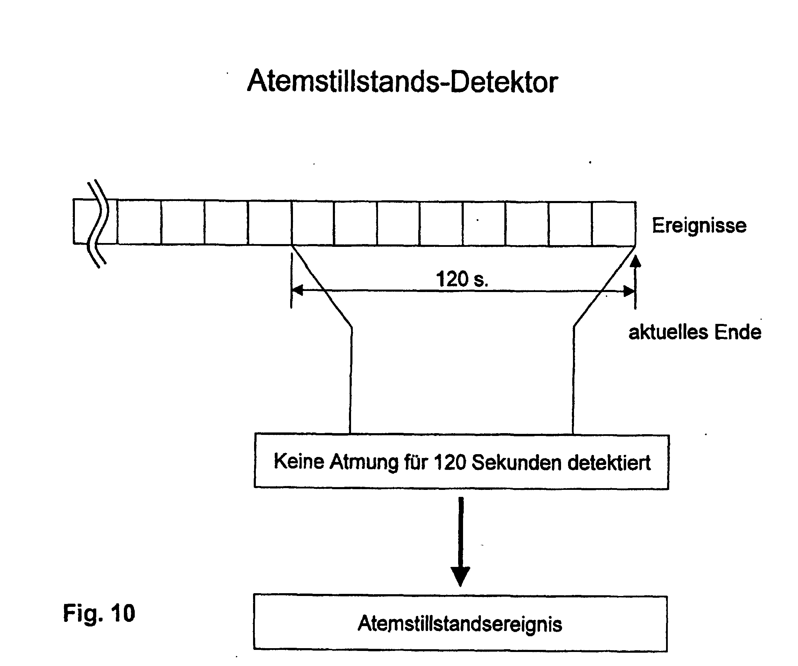

Der Atemstillstanddetektor (Figur 1, Bezugszeichenll) generiert den Zustand eines Atemstillstands anzeigende Signal S6. In der Figur 10 ist die Funktionsweise des Atemstillstandsdetektors schematisch dargestellt. Dieser wird aktiviert, wenn nach einer bestimmten Zeit beispielsweise 120 Sekunden keine Atmung auf Grundlage der Atemzugserkennung (Figur 1, Bezugszeichenerkannt 12) erkannt wird.

### Hypopnoe-Detektor

Der Hypopnoe-Detektor (Figur 1, Bezugszeichen 4) generiert den Zustand einer Hypopnoe-Phase anzeigende Signal S2 auf Grundlage des mittleren Inspirationsvolumens sowie unter Rückgriffnahme auf Ergebnisse ( Signale/Daten Informationen B2) der Rückwärtskorrelationsanalyse.

Ein Hypopnoe-Zustand wird erkannt, wenn innerhalb einer Serie mit einer vorgegebenen Anzahl an Atemzügen, vorzugsweise sechs Atemzügen, gegenüber einem mittleren Atemzugsvolumen einer Abweichung vorliegt, die ein Schwellwert-Kriterium erfüllt. Bei diesem Schwellwertkriterium können mehrere Schwellwerte berücksichtigt werden. Unterschieden werden zwei Arten von Hypopnoe-Zustände, der stabilen und der kritischen Hypopnoe.

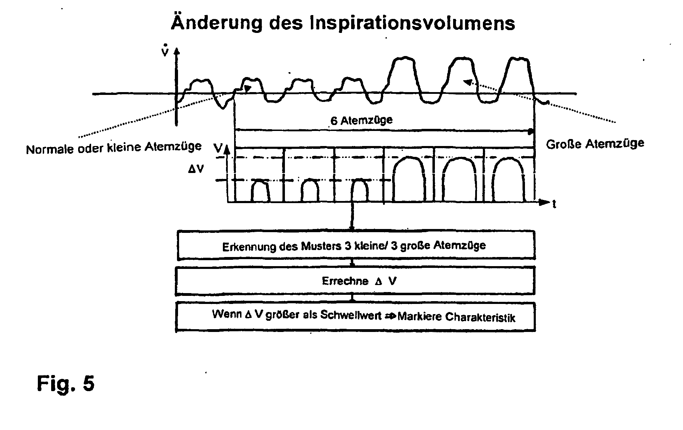

Bei der Generierung eines Hypopnoe-Zustand erfolgt insbesondere hinsichtlich der Betrachtung der Atemzugsvolumina, eine Betrachtung der Atemgasflusskurve, wie sie in Figur 5 verdeutlicht ist. Der in dieser Darstellung oberste Graph zeigt den zeitlichen Verlauf des Atemgasflusses. Der darunterliegende Graph zeigt den stark geglätteten Verlauf derartiger Atemzüge. Im Rahmen einer Atemmustererkennung werden vorzugsweise drei kleinere und drei größere Atemzüge erkannt.

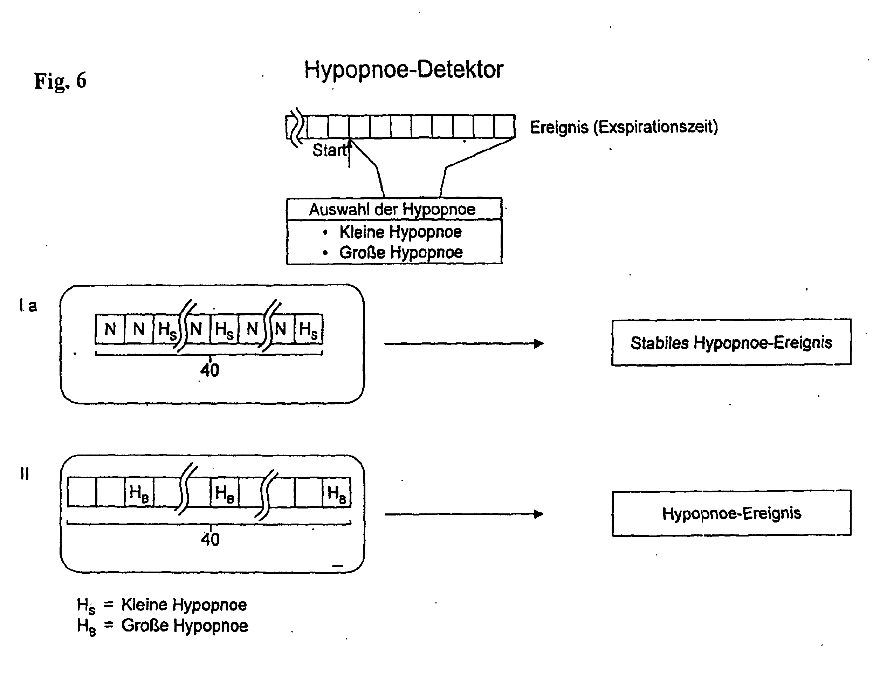

In Figur 6 ist diesbezüglich ein Diagramm dargestellt, nach welchem ein stabiles Hypopnoe-Ereignis dann als vorliegend angesehen wird, wenn innerhalb einer Serie von 40 Atemzügen vorzugsweise drei kleinere Hypopnoen mit mehr als 1800 ml Inspirationsvolumen erkannt wurden. Bei der Generierung des den stabilen Hypopnoe-Zustand klassifizierenden Signal S2 erfolgt weiterhin eine Bewertung der Rückwärtskorrelationsbetrachtung und weisen vorzugsweise mindestens eine Korrelation von 0,86 auf.

Ein kritisches Hypopnoe-Ereignis wird erkannt, wenn innerhalb einer Serie von 40 Atemzügen vorzugsweise drei große Hypopnoen mit mehr als 3600 ml Inspirationsvolumen erkannt wurde und generiert den Zustand einer Hypopnoe angebendes Signale S2. Eine zusätzliche Bewertung kann durch die Korrelation erfolgen.

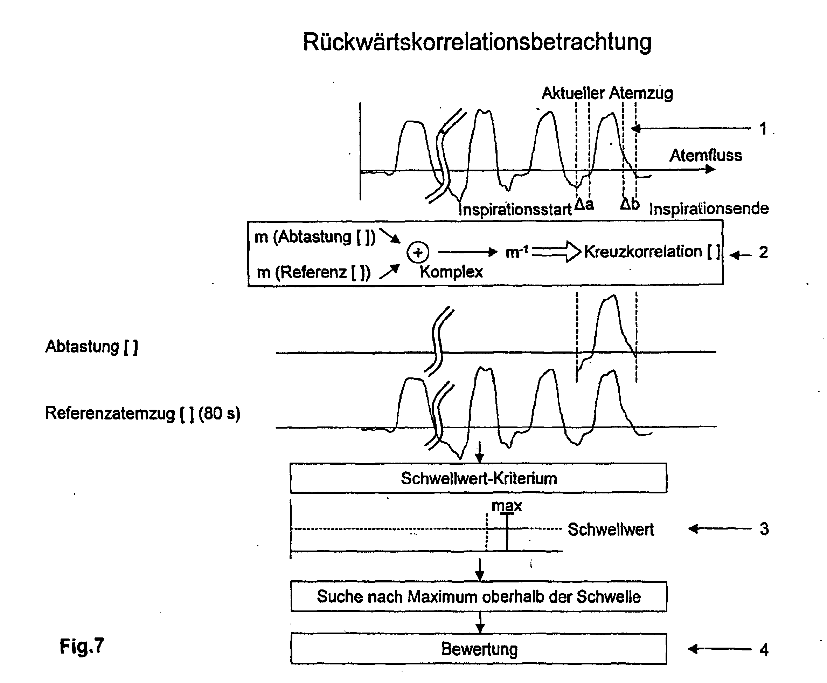

Die Systematik einer bevorzugten Ausführungsform der Rückwärtskorrelationsbetrachtung ist in Figur 7 verdeutlicht. Bei der Rückwärtskorrelationsbetrachtung wird die Phase der Inspiration ausgewertet. Der Wert der Kreuzkorrelation (Figur 7, Bezugszeichen 2) wird aus dem aktuellen inspiratorischen Atemzug und der vorzugsweise 40 letzten inspiratorischen Atemzüge berechnet. Ein zusätzliches Schwellwert-Kriterium bewertet den Wert der Rückwärtskorrelation jedes Atemzugs (Figur 7, Bezugszeichen 3,4) vorzugsweise für die Hypopnoe-, Krümmungs- und Normalatmungs-Detektor.

### Schnarchbonus

Der in der Figur 1 durch das Bezugszeichen 21 gekennzeichnete Schnarchbonus erzeugt das indikative Signal P1.

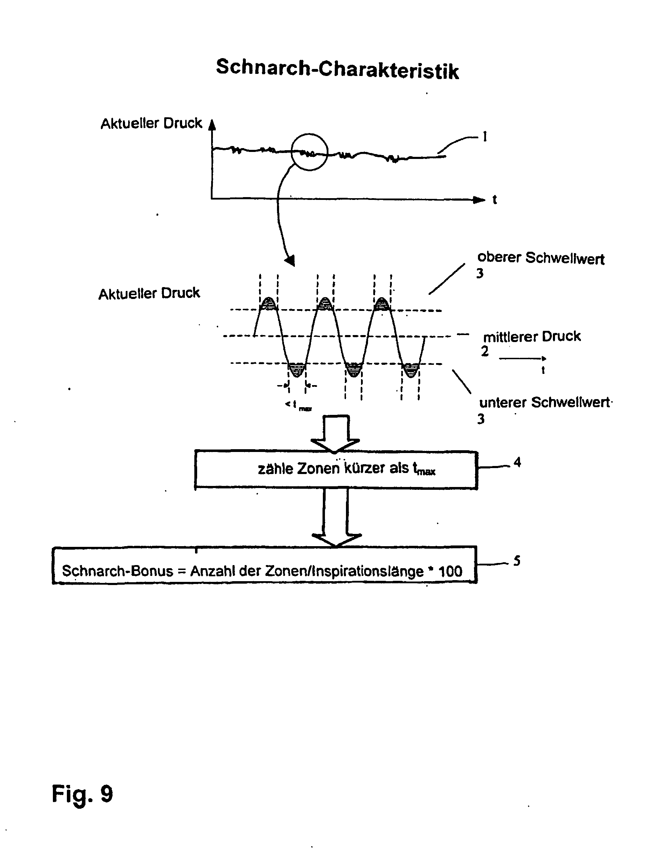

Figur 9 zeigt schematisch, wie der Schnarchbonus aus dem indikativen Signal P3 (Figur 9, Bezugszeichen 1) gewonnen wird. Für die Berechnung des Schnarchbonus wird aus dem aktuellen Drucksignal P3 der Mittelwert (Figur 9, Bezugszeichen 2) gebildet. Dieser aktuelle Mittelwert wird als Bezugslinie für die Schwellwerte (Figur 9, Bezugszeichen 3) genommen. Bei Schnarchen entsteht auf dem aktuellen Drucksignal P3 eine Oszillation. Hierbei muss die Oszillation eine Schwelle überschreiten beispielsweise 0,1 bzw. 0,5. Bei Überschreitung der Schwellwerte erfolgt eine Zeitanalyse (Figur 9, Bezugszeichen 4), um spezifische Frequenzanteile auszuschließen. Berechnet wird der Schnarchbonus vorzugsweise (Figur 9, Bezugszeichen 5) aus dem Quotient der Nulldurchgänge (Oszillation) und der Länge der Inspiration. Der Quotient wird anschließend mit vorzugsweise 100 multipliziert.

Eine Besonderheit dieser Methode ist die Feststellung des Schnarchen mit der Phase der Atmung. Mit Hilfe des indikativen Signals V1 und dem indikativen Signal P3 kann unterschieden werden ob ein Schnarchen während der Inspiration, Exspiration sowie der gesamten Atmung erfolgt. Ausgewertet wird vorzugsweise Schnarchen während der Inspirationsphase. Tritt auf dem indikativen Signal P3 Schnarchen auf, verändert der Schnarchbonus die Schwellwerte (indikatives Signal P1; Figur 1, Bezugszeiten P1) für den Krümmungsdetektor (Figur 1, Bezugszeichen 9).

### Krümmungsdetektor

Der in Figur 1 durch das Bezugszeichen 9 gekennzeichnete Krümmungsdetektor kann das Bewertungssignal S3 und S4 auf Grundlage des hinsichtlich des Krümmungsbestimmungs-Modules 13 (indikatives Signal B4), Rückwärtskorrelation 7 (indikatives Signal B3) und des Schnarchbonus 21 (indikatives Signal P1) erzeugen werden.

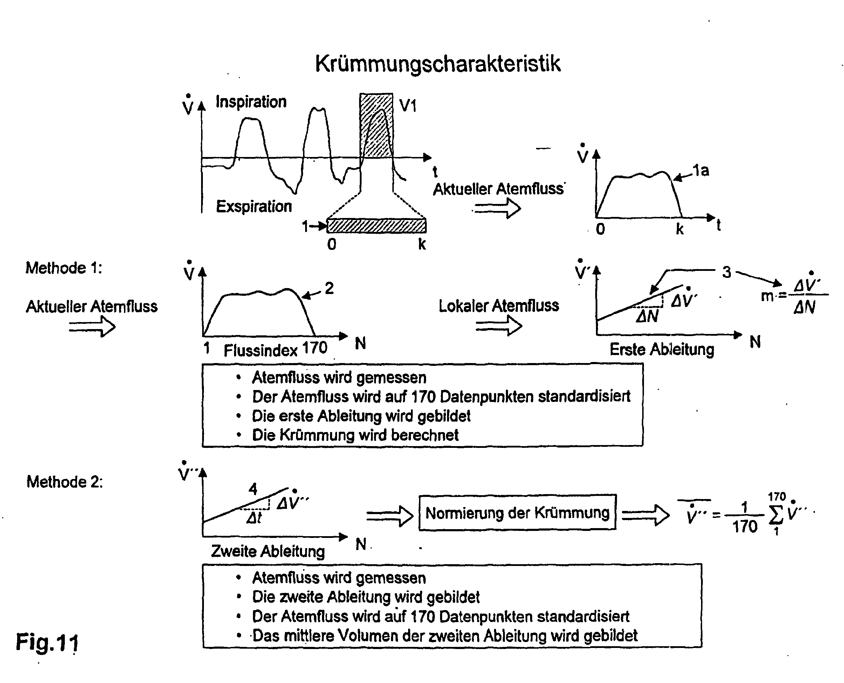

In der Figur 11 wird die Funktionsweise des Krümmungsdetektors schematisch erklärt. Die Krümmung kann durch zwei Methoden berechnet werden:

### 1. Methode:

Aus dem aktuellen Atemflusssignal V1 (Figur 1 und Figur 11, Bezugszeichen V1) wird die Phase der Inspiration und/oder der Exspiration ermittelt. Da die Phase des Atemzugs eine beliebige Anzahl von Datenpunkten k (Figur 11, Bezugszeichen 1 und 1a) besitzt, werden die inspiratorischen und/oder die exspiratorischen Datenpunkten über einen Flowindex von vorzugsweise 1 bis 170 normiert (Figur 11, Bezugszeichen 2). Aus diesem normierten inspiratorischen und/oder exspiratorischen Atemflusssignal wird die erste Ableitung gebildet. Ausgewertet wird der Wert der Steigung m (Figur 11, Bezugszeichen 3) aus dem Quotient der ersten Ableitung des Atemflussdeltas und des Deltas der normierten Zeit. Der berechnete Quotient entspricht der mittleren Krümmung (zweite Ableitung des Atemfluss V1).

### 2. Methode

Aus dem aktuellen Atemflusssignal V1 (Figur 1 und Figur 11, Bezugszeichen V1) wird die Phase der Inspiration und/oder der Exspiration ermittelt. Aus der Phase des Atemzugs wird die zweite Ableitung gebildet (Figur 11, Bezugszweichen 4). Anschießend werden die berechneten Krümmungen der inspiratorischen bzw. exspiratorischen Phasen vorzugsweise zwischen 1 und 170 normiert (Figur 11, Bezugszeichen 5) und der Mittelwert (Figur 11, Bezugszeichen 6) gebildet.

Berechnet sich aus dem Atemflusssignal V1 (Figur 11, Bezugszeichen V1) einen Wert der mittleren Krümmung beispielweise >0,5, ist diese ein Indiz für eine pathologische Veränderung des Atemflusses wie Flusslimitierung.

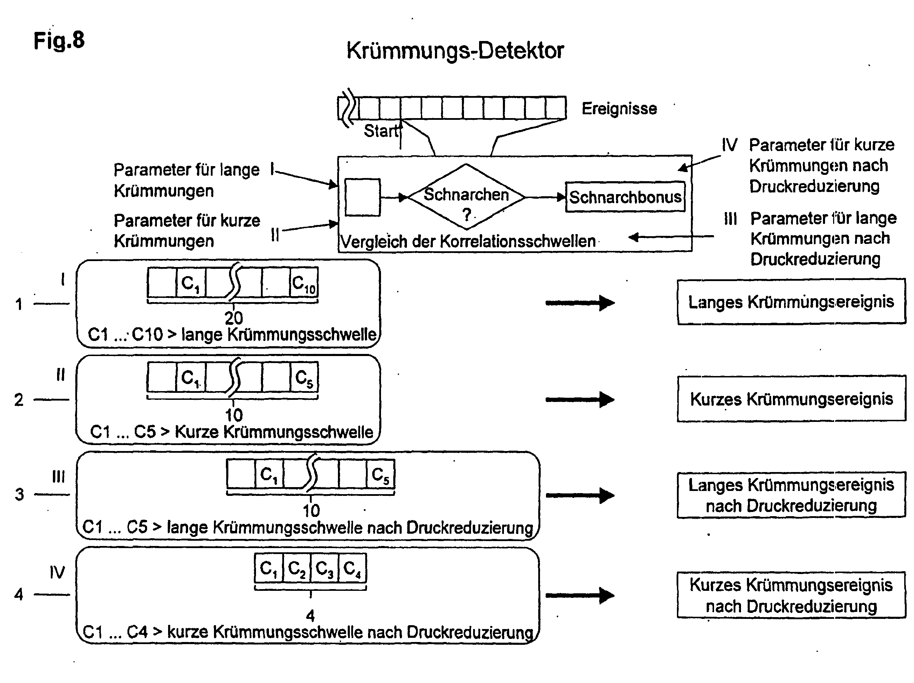

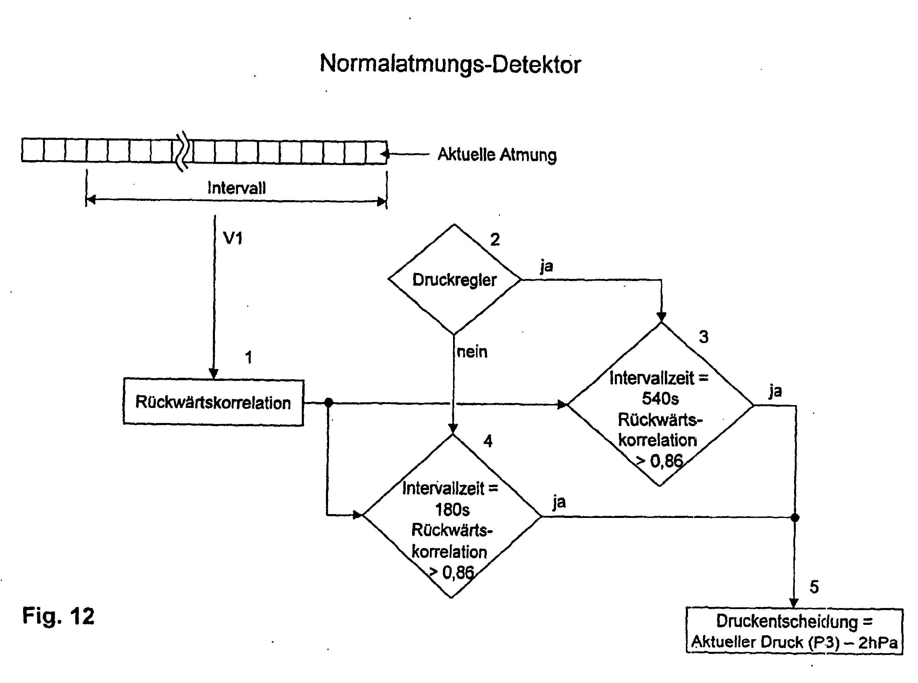

Bei der Generierung der den Krümmungsdetektors klassifizierenden Signalen S3 und S4 erfolgt weiterhin eine Bewertung der Rückwärtskorrelationsbetrachtung und des Schnarchbonus durchgeführt. Der Krümmungsdetektor unterscheidet zwischen vier auftretenden Ereignissen:
1. langes Krümmungsereignis (Figur 8, Bezugszeichen 1):
   Dieses Ereignis existiert vorzugsweise, wenn eine mittlere Rückrückwärtskorrelation und 10 von 20 Atemzügen eine mittlere Krümmung von beispielsweise >0,5 detektiert wurde. Tritt zusätzlich Schnarchen auf, wird der Schwellwert der Krümmung erniedrigt und das System somit selektiver. Dieses Ereignis ist nur für die Erhöhung des Therapiedrucks (Figur 1, Bezugszeichen P2) aktiv.
2. kurzes Krümmungsereignis (Figur 8, Bezugszeichen 2):
   Dieses Ereignis existiert vorzugsweise, wenn eine Rückrückwärtskorrelation > 0,86 und 5 von 10 Atemzügen eine mittlere Krümmung von beispielsweise > 0,5 detektiert wurde. Tritt zusätzlich Schnarchen auf, wird der Schwellwert der Krümmung erniedrigt und das System somit selektiver.
3. langes Krümmungsereignis nach Druckreduzierung (Figur 12, Bezugszeichen 3):
   Dieses Ereignis existiert vorzugsweise, wenn der Therapiedruck (Figur 1, Bezugszeichen P2) erniedrigt wird. 5 aus 10 Atemzügen muss die mittlere Krümmung von beispielsweise >0,5 detektiert werden. Die Rückwärtskorrelation und der Schnarchbonus kann zusätzliche in die Bewertung dieses Signals eingehen.
4.kurzes Krümmungsereignis nach Druckreduzierung (Figur 12, Bezugszeichen 4). Dieses Ereignis existiert vorzugsweise, wenn der Therapiedruck (Figur 1, Bezugszeichen P2) erniedrigt wird. 4 aus 4 Atemzügen muss die mittlere Krümmung von beispielsweise >0,5 detektiert werden. Die Rückwärtskorrelation und der Schnarchbonus kann zusätzliche in die Bewertung dieses Signals eingehen.

### Normal-Atmungsdetektor

Der Normal-Atmungsdetektor (Figur 1, Bezugszeichen 10) generiert den Zustand einer Normalatem-Phase anzeigende Signal S5 auf Grundlage der Rückwärtskorrelation (Figur 1, Bezugszeichen 7).

In der Figur 12 ist der Normal-Atmungsdetektor schematisch dargestellt. Das Atemflusssignal V1 wird durch die Rückwärtskorrelation (Figur 1, Bezugszeichen 7; Figur 12 Bezugszeichen 1) bewertet. Über den momentanen Schaltzustand der Steuereinrichtung (Figur 12, Bezugszeichen 2) wird die Länge des Auswerteintervalls bestimmt. Die Steuereinrichtung unterscheidet zwischen normaler und sensitiver Druckregelung. Ist die Steuereinrichtung im Betriebsmodus "normaler Druckregelung", ist die Länge des Auswerteintervalls vorzugsweise 540 Sekunden; bei "sensitiver Drucksteuerung" ist das Auswerteintervall vorzugsweise 180 Sekunden (Figur 12, Bezugszeichen 4). Aktiviert wird der Normal-Atmungsdetektor, wenn innerhalb des Auswerteintervalls der Atemfluss vorzugsweise eine Rückwärtskorrelation von mindestens 0,86 beträgt. Der Normal-Atmungsdetektor wird vorzugsweise für die Druckreduzierung des Systems verwendet.

### Leckagedetektor

Der in Figur 1 durch das Bezugszeichen 18 gekennzeichnete Leckagedetektor kann das Bewertungssignal S7 auf Grundlage des hinsichtlich der Atemzugsdetektion 12 (indikatives Signal V1) und des aktuell gemessen Drucks P3 (indikatives Signal P3) ermitteltn.

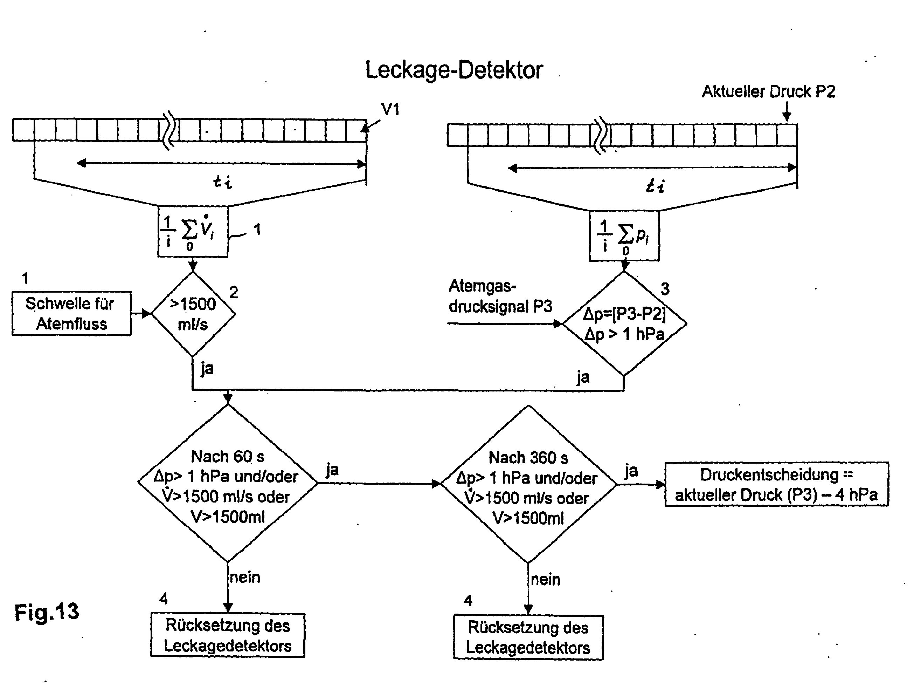

In der Figur 13 ist die Funktionsweise des Leckagedetektors schematisch dargestellt. Die Leckage-Erkennung kann über zwei Methoden erfolgen, über die Auswertung des Atemflusses und/oder über den Druck. Im folgenden werden die zwei Methoden beschrieben:
1. Leckageerkennung über das Atemflusssignal:
   Aus dem Atemflusssignal V1 wird über ein Zeitintervall von beispielsweise 60 sec ein Mittelwert (Figur 13, Bezugszeichen 1) gebildet. Überschreitet der Mittelwert eine bestimmte Schwelle (Figur 13, Bezugszeichen 2), beispielsweise 1500 ml/sec, wird die sensitive und normale Drucksteuerung über den Schalter C (Figur 1, Bezugszeichen C) deaktiviert. Befindet sich der Mittelwert nach beispielsweise 360 Sekunden immer noch über den Schwellwert, wird der CPAP-Druck um 4 hPa erniedrigt, bis maximal bis auf einen Mindestdruck von 4 hPa. Alternativ kann für die Erkennung der Atemflussleckage auch das Volumen des Atemflusses berechnet und ausgewertet werden.
2. Leckageerkennung über den Druck:
   Aus den Drucksignalen des Solldrucks P2 und dem aktuellen Istdruck P3 wird die Abweichung (Figur 13, Bezugszeichen 3) berechnet. Beträgt die Differenz vorzugsweise 1hPa wird die sensitive und normale Drucksteuerung über den Schalter C (Figur 1, Bezugszeichen C) deaktiviert. Ist die Abweichung nach beispielsweise 360 Sekunden immer noch über 1 hPa, wird der CPAP-Druck um 4 hPa erniedrigt, maximal bis auf einen Mindestdruck von 4 hPa.
   Ist die Leckageerkennung aktiv, führen die detektierten Ereignisse zu keine Druckveränderung, da die sensitive und normale Drucksteuerung nicht aktiv ist. Werden die Schwellwerte für den Atemfluss (1500 ml/sec oder Differenzdruck 1hPa) unterschritten, wird die Drucksteuerung erst nach einer Zeit von beispielsweise 30 Sekunden aktiv und Variablen neu gesetzt (Figur 13, Bezugszeichen 4).

Die erfindungsgemäße Steuerungseinrichtung ist vorzugsweise als elektronische Schaltung mit speicherprogrammierbaren Elementen, aufgebaut. Die Einstellung des Atemgasdruckes nach Maßgabe des erfindungsgemäß ermittelten Atemgassolldruckes erfolgt vorzugsweise durch eine Regelkreis mit hoher Regeldynamik. Die Einstellung des Atemgasdruckes nach Maßgabe des Atemgas-Solldruckes erfolgt vorzugsweise durch Regelung der Förderleistung einer Gebläseeinrichtung beispielsweise durch Änderung der Drehzahl eines Laufrades der Gebläseeinrichtung. Alternativ hierzu - oder auch in Kombination mit dieser Maßnahme ist es auch möglich, den Atemgasdruck über Ventileinrichtungen oder Drosselorgane auf den vorgegebenen Atemgas-Solldruck einzustellen.

## Patentansprüche

1. Steuerungseinrichtung zur Bestimmung eines Atemgassolldruckes zur Diagnose und/oder Therapie schlafbezogener Atmungsstörungen mit:
einem Flusssignalgenerator zur Generierung eines hinsichtlich des Atemgasflusses indikativen Atemgas-Flusssignales,
einem Drucksignalgenerator zur Generierung eines hinsichtlich des Atemgasdruckes indikativen Atemgasdrucksignales, und
einer Signalverarbeitungseinrichtung zur Verarbeitung des Atemgas-Flussignales,
**dadurch gekennzeichnet, dass** die Signalverarbeitungseinrichtung derart ausgebildet ist, dass durch diese auf Grundlage des Atemgas-Flusssignales ein Spezifikationssignal generiert wird, wobei in Abhängigkeit von dem Spezifikationssignal ein zur Einstellung eines Atemgasdruckes relevantes Druckvorgabesignal nach Maßgabe eines ersten Druckermittlungamodus oder nach Maßgabe eines von dem ersten Druckermittlungsmodus, zumindest hinsichtlich seines Zeitverhaltens abweichenden zweiten Druckermittlungsmodus generiert wird.

2. Steuerungseinrichtung nach Anspruch 1, **dadurch gekennzeichnet, dass** das Spezifikationssignal durch Auswertung des Atemgas-Flusssignales auf Grundlage mehrerer Kriterien generiert wird.

3. Steuerungseinrichtung nach Anspruch 2, **dadurch gekennzeichnet, dass** im Zuge der Auswertung des Atemgas-Flusssignales für jedes Kriterium wenigstens ein Bewertungssignal generiert wird, das die Erfüllung des Kriteriums beschreibt.

4. Steuerungseinrichtung nach Anspruch 3, **dadurch gekennzeichnet, dass** zur Generierung des Spezifikationssignals mehrere Bewertungssignale gemeinsam betrachtet werden.

5. Steuerungseinrichtung nach Anspruch 3 oder 4, **dadurch gekennzeichnet, dass** wenigstens eines der Bewertungssignale durch eine Apnoe-Detektionseinrichtung generiert wird.

6. Steuerungseinrichtung nach wenigstens einem der Ansprüche 3 bis 5, **dadurch gekennzeichnet, dass** eines der Bewertungssignale durch eine Hypopnoe-Detektionseinrichtung generiert wird.

7. Steuerungseinrichtung nach wenigstens einem der Ansprüche 3 bis 6, **dadurch gekennzeichnet, dass** wenigstens eines der Bewertungssignale durch eine Krümmungs-Beurteilungseinrichtung generiert wird.

8. Steuerungseinrichtung nach wenigstens einem der Ansprüche 3 bis 7, **dadurch gekennzeichnet, dass** wenigstens eines der Bewertungssignale durch eine Normalatmungs-Detektionseinrichtung generiert wird.

9. Steuerungseinrichtung nach wenigstens einem der Ansprüche 3 bis 8, **dadurch gekennzeichnet, dass** das wenigstens eine der Bewertungssignale durch eine Atemstillstands-Detektionseinrichtung generiert wird.

10. Steuerungseinrichtung nach wenigstens einem der Ansprüche 1 bis 9, **dadurch gekennzeichnet, dass** eine Leckage-Detektionseinrichtung vorgesehen ist zur Erfassung eines Leckagezustandes; wobei im Falle erkannter Leckage die Druckregelung mittels einer Leckage-Druckregeleinrichtung erfolgt.

11. Steuerungseinrichtung nach wenigstens einem der Ansprüche 1 - 10, **dadurch gekennzeichnet, dass** eine Atemzugslängen-Erfassungseinrichtung vorgesehen ist zur Generierung eines hinsichtlich der zeitlichen Länge eines Atemzyklus indikativen Atemzykluszeitdauer-Signals.

12. Steuerungseinrichtung nach wenigstens einem der Ansprüche 1 - 11, **dadurch gekennzeichnet, dass** eine Expirations-Zeitdauererfassungseinrichtung vorgesehen ist zur Generierung eines hinsichtlich der zeitlichen Länge einer Expirationsphase indikativen Signales.

13. Steuerungseinrichtung nach wenigstens einem der Ansprüche 1 - 12, **dadurch gekennzeichnet, dass** eine Inspirations-Zeitdauererfassungseinrichtung vorgesehen ist zur Generierung eines hinsichtlich der zeitlichen Länge einer Inspirationsphase indikativen Signales.

14. Steuerungseinrichtung nach wenigstens einem der Ansprüche 1 - 13, **dadurch gekennzeichnet, dass** eine Volumenerfassungseinrichtung vorgesehen ist zur Erfassung eines Atemzugsvolumens.

15. Steuerungseinrichtung nach wenigstens einem der Ansprüche 1 - 14, **dadurch gekennzeichnet, dass** eine Korrelationserfassungseinrichtung vorgesehen ist zur Erfassung einer Rückwärtskorrelationsbeziehung des momentanen Atemzuges mit einem hinsichtlich zurückliegender Atemzüge repräsentativen Atemzugverlaufes.

16. Steuerungseinrichtung nach wenigstens einem der Ansprüche 1 - 15, **dadurch gekennzeichnet, dass** eine Krümmungsermittlungseinrichtung vorgesehen ist zur Ermittlung der Krümmung des Atemgasstromverlaufes.

17. Steuerungseinrichtung nach wenigstens einem der Ansprüche 1 - 16, **dadurch gekennzeichnet, dass** die Apnoe-Detektionseinrichtung das Schaltsignal auf Grundlage des seitens der Expirations-Zeitdauererfassungseinrichtung erzeugten Signales generiert.

18. Steuerungseinrichtung nach wenigstens einem der Ansprüche 1 bis 17, **dadurch gekennzeichnet, dass** die Hypopnoe-Detektionseinrichtung das Bewertungssignal auf Grundlage des durch die Volumenerfassungseinrichtung determinierten Atemzugvolumens und der durch die Korrelationserfassungseinrichtung determinierten Rückwärtskorrelationsbeziehung generiert.

19. Steuerungseinrichtung nach wenigstens einem der Ansprüche 1 bis 18, **dadurch gekennzeichnet, dass** die Normalatmungs-Detektionseinrichtung das Bewertungssignal auf Grundlage des Auswertungsergebnisses der Rückwärtskorrelationsbeziehung generiert.

20. Steuerungseinrichtung nach wenigstens einem der Ansprüche 1 bis 19, **dadurch gekennzeichnet, dass** die Atemstillstands-Detektionseinrichtung das Bewertungssignal auf Grundlage des Atemgas-Flusssignales generiert.

21. Steuerungseinrichtung nach wenigstens einem der Ansprüche 1 bis 20, **dadurch gekennzeichnet, dass** eine Schnarcherkennungseinrichtung vorgesehen ist zur Generierung eines ein Schnarchereignis klassifizierenden Signales.

22. Steuerungseinrichtung nach wenigstens einem der Ansprüche 1 bis 21, **dadurch gekennzeichnet, dass** die Schnarcherkennungseinrichtung das Schnarchereignis auf Grundlage einer Null-Durchgangsbetrachtung der ersten Ableitung des Atemgasdruckes klassifiziert.

23. Steuerungseinrichtung nach wenigstens einem der Ansprüche 1 bis 22, **dadurch gekennzeichnet, dass** die Ausführung einer Umschaltung zwischen den wenigstens zwei Drucksteuerungsmodi auf Grundlage einer Auswertung mehrerer Bewertungssignale erfolgt.

24. Steuerungseinrichtung nach wenigstens einem der Ansprüche 1 bis 23, **dadurch gekennzeichnet, dass** die Bewertungssignale mit Gewichtungsfaktoren gewichtet berücksichtigt werden.

25. Steuerungseinrichtung nach wenigstens einem der Ansprüche 1 bis 24, **dadurch gekennzeichnet, dass** die Gewichtungsfaktoren auf Grundlage von Bewertungen festgelegt werden, die aus dem hinsichtlich des Atemgasstromes indikativen Signal extrahiert werden.

26. Steuerungseinrichtung nach Anspruch 24 oder 25, **dadurch gekennzeichnet, dass** die Gewichtungsfaktoren in Abstimmung zueinander festgelegt werden.

27. Steuerungseinrichtung nach wenigstens einem der Ansprüche 24 bis 26, **dadurch gekennzeichnet, dass** die zeitliche Änderung der Gewichtungsfaktoren durch ein Dämpfungssystem gedämpft ist.

28. Steuerungseinrichtung nach wenigstens einem der Ansprüche 24 bis 27, **dadurch gekennzeichnet, dass** der erste Druckermittlungsmodus auf ein Hypopnoe-Ereignis bei einem Schwellwert anspricht, der höher ist, als der korrespondierende Schwellwert im zweiten Druckermittlungsmodus.

29. Steuerungseinrichtung nach wenigstens einem der Ansprüche 1 bis 28, **dadurch gekennzeichnet, dass** in dem zweiten Druckermittlungsmodus der Atemgasdruck mit einer höheren Regeldynamik angepasst wird.

## Revendications

1. Dispositif de commande pour définir une pression de gaz respiratoire de consigne pour le diagnostic et/ou la thérapeutique de troubles respiratoires durant le sommeil, comportant:
un générateur de signal de flux pour générer un signal de flux de gaz respiratoire indicatif du flux de gaz respiratoire,
un générateur de signal de pression pour générer un signal de pression de gaz respiratoire indicatif de la pression de gaz respiratoire et
un dispositif de traitement de signaux pour traiter le signal de flux de gaz respiratoire,
**caractérisé en ce que** le dispositif de traitement de signaux est configuré de manière à ce qu'il génère un signal de spécification sur la base du signal de flux de gaz respiratoire, un signal de consigne de la pression pertinente pour le réglage d'une pression de gaz respiratoire étant généré en fonction du signal de spécification déterminé d'après un premier mode de détermination de la pression ou déterminé d'après un second mode de détermination de la pression qui s'écarte du premier mode de détermination de la pression du moins par son comportement dans le temps.

2. Dispositif de commande selon la revendication 1, **caractérisé en ce que** le signal de spécification est généré par le dépouillement du signal de flux de gaz respiratoire sur la base de plusieurs critères.

3. Dispositif de commande selon la revendication 2, **caractérisé en ce qu'**au gré du dépouillement du signal de flux de gaz respiratoire au moins un signal d'évaluation est généré pour chaque critère, qui décrit l'accomplissement du critère.

4. Dispositif de commande selon la revendication 3, **caractérisé en ce que** pour générer le signal de spécification plusieurs signaux d'évaluation sont considérés ensemble.

5. Dispositif de commande selon la revendication 3 ou 4, **caractérisé en ce qu'**au moins un des signaux d'évaluation est généré par un dispositif de détection d'apnée.

6. Dispositif de commande selon au moins l'une des revendications 3 à 5, **caractérisé en ce qu'**un des signaux d'évaluation est généré par un dispositif de détection d'hypopnée.

7. Dispositif de commande selon au moins l'une des revendications 3 à 6, **caractérisé en ce qu'**au moins un des signaux d'évaluation est généré par un dispositif d'évaluation de la courbure.

8. Dispositif de commande selon au moins l'une des revendications 3 à 7, **caractérisé en ce qu'**au moins un des signaux d'évaluation est généré par un dispositif de détection de la respiration normale.

9. Dispositif de commande selon au moins l'une des revendications 3 à 8, **caractérisé en ce que** le au moins un des signaux d'évaluation est généré par un dispositif de détection de l'arrêt respiratoire.

10. Dispositif de commande selon au moins l'une des revendications 1 à 9, **caractérisé en ce qu'**un dispositif de détection d'une fuite est prévu pour saisir un état de fuite, en cas d'une fuite détectée le réglage de la pression se faisant au moyen d'un dispositif de réglage de la pression en cas de fuite.

11. Dispositif de commande selon au moins l'une des revendications 1 à 10, **caractérisé en ce qu'**un dispositif de saisie de la longueur d'une respiration est prévu pour générer un signal de durée du cycle respiratoire indicatif de la longueur dans le temps d'un cycle respiratoire.

12. Dispositif de commande selon au moins l'une des revendications 1 à 11, **caractérisé en ce qu'**un dispositif de saisie de la durée d'une expiration est prévu pour générer un signal indicatif de la longueur dans le temps d'une phase d'expiration.

13. Dispositif de commande selon au moins l'une des revendications 1 à 12, **caractérisé en ce qu'**un dispositif de saisie de la durée d'inspiration est prévu pour générer un signal indicatif de la longueur dans le temps d'une phase d'inspiration.

14. Dispositif de commande selon au moins l'une des revendications 1 à 13, **caractérisé en ce qu'**un dispositif de saisie du volume est prévu pour saisir le volume d'une respiration.

15. Dispositif de commande selon au moins l'une des revendications 1 à 14, **caractérisé en ce qu'**un dispositif de saisie de la corrélation est prévu pour saisir un rapport de corrélation à rebours de la respiration momentanée avec une évolution de respirations représentative de respirations antérieures.

16. Dispositif de commande selon au moins l'une des revendications 1 à 15, **caractérisé en ce qu'**un dispositif de détermination de la courbure est prévu pour déterminer la courbure de l'évolution du flux de gaz respiratoire.

17. Dispositif de commande selon au moins l'une des revendications 1 à 16, **caractérisé en ce que** le dispositif de détection d'apnée génère le signal de commutation sur la base du signal produit par le dispositif de saisie de la durée de l'expiration.

18. Dispositif de commande selon au moins l'une des revendications 1 à 17, **caractérisé en ce que** le dispositif de détection d'hypopnée génère le signal d'évaluation sur la base du volume de respiration déterminé par le dispositif de saisie du volume et du rapport de corrélation à rebours déterminé par le dispositif de saisie de la corrélation.

19. Dispositif de commande selon au moins l'une des revendications 1 à 18, **caractérisé en ce que** le dispositif de détection de la respiration normale génère le signal d'évaluation sur la base du résultat de dépouillement du rapport de corrélation à rebours.

20. Dispositif de commande selon au moins l'une des revendications 1 à 19, **caractérisé en ce que** le dispositif de détection de l'arrêt respiratoire génère le signal d'évaluation sur la base du signal du flux de gaz respiratoire.

21. Dispositif de commande selon au moins l'une des revendications 1 à 20, **caractérisé en ce qu'**un dispositif de détection du ronflement est prévu pour générer un signal de classification de l'événement de ronflement.

22. Dispositif de commande selon au moins l'une des revendications 1 à 21, **caractérisé en ce que** le dispositif de détermination du ronflement classe l'événement de ronflement sur la base d'une observation d'un passage à zéro de la première déviation de la pression de gaz respiratoire.

23. Dispositif de commande selon au moins l'une des revendications 1 à 22, **caractérisé en ce que** l'exécution d'une commutation entre les modes de commande de la pression, étant au moins au nombre de deux, se fait sur la base d'un dépouillement de plusieurs signaux d'évaluation.

24. Dispositif de commande selon au moins l'une des revendications 1 à 23, **caractérisé en ce que** les signaux d'évaluation sont pris en compte pondérés par des facteurs de pondération.

25. Dispositif de commande selon au moins l'une des revendications 1 à 24, **caractérisé en ce que** les facteurs de pondération sont déterminés sur la base d'évaluations qui sont extraites du signal indicatif du flux de gaz respiratoire.

26. Dispositif de commande selon la revendication. 24 ou 25, **caractérisé en ce que** les facteurs de pondération sont déterminés en correspondance réciproque.

27. Dispositif de commande selon au moins l'une des revendications 24 à 26, **caractérisé en ce que** la modification dans le temps des facteurs de pondération est amortie par un système d'amortissement.

28. Dispositif de commande selon au moins l'une des revendications 24 à 27, **caractérisé en ce que** le premier mode de détermination de la pression réagit à un événement d'hypopnée à une valeur de seuil qui est supérieure à la valeur de seuil correspondante dans le second mode de détermination de la pression.

29. Dispositif de commande selon au moins l'une des revendications 1 à 28, **caractérisé en ce que** dans le second mode de détermination de la pression, la pression de gaz respiratoire est adaptée au moyen d'une dynamique de régulation plus élevée.

## Claims

1. A control apparatus for determining a respiratory gas desired pressure for diagnosis and/or therapy of sleep-related respiratory disorders, comprising:
a flow signal generator for generating a respiratory gas flow signal indicative in respect of the respiratory gas flow,
a pressure signal generator for generating a respiratory gas pressure signal indicative in respect of the respiratory gas pressure, and
a signal processing device for processing the respiratory gas flow signal,
**characterised in that** the signal processing device is so designed that a specification signal is generated thereby on the basis of the respiratory gas flow signal, wherein in dependence on the specification signal a pressure presetting signal which is relevant for adjustment of a respiratory gas pressure is generated in accordance with a first pressure-determination mode or in accordance with a second pressure-determination mode which differs from the first pressure-determination mode at least in regard to its time characteristics.

2. A control apparatus according to claim 1 **characterised in that** the specification signal is generated by assessment of the respiratory gas flow signal on the basis of a plurality of criteria.

3. A control apparatus according to claim 2 **characterised in that** in the course of assessment of the respiratory gas flow signal for each criterion at least one evaluation signal is generated, which describes fulfilment of the criterion.

4. A control apparatus according to claim 3 **characterised in that** a plurality of evaluation signals are considered jointly for generation of the specification signal.

5. A control apparatus according to claim 3 or claim 4 **characterised in that** at least one of the evaluation signals is generated by an apnoea detection device.

6. A control apparatus according to at least one of claims 3 to 5 **characterised in that** one of the evaluation signals is generated by a hypopnoea detection device.

7. A control apparatus according to at least one of claims 3 to 6 **characterised in that** at least one of the evaluation signals is generated by a curvature evaluation device.

8. A control apparatus according to at least one of claims 3 to 7 **characterised in that** at least one of the evaluation signals is generated by a normal respiration detection device.

9. A control apparatus according to at least one of claims 3 to 8 **characterised in that** the at least one of the evaluation signals is generated by a respiration stoppage detection device.

10. A control apparatus according to at least one of claims 1 to 9 **characterised in that** there is provided a leakage detection device for detection of a leakage condition, wherein in the case of detected leakage pressure regulation is effected by means of a leakage pressure regulating device.

11. A control apparatus according to at least one of claims 1 to 10 **characterised in that** there is provided a breath length detection device for generation of a breath cycle time duration signal indicative in respect of the length in respect of time of a breath cycle.

12. A control apparatus according to at least one of claims 1 to 11 **characterised in that** there is provided an expiration time duration detection device for the generation of a signal indicative in respect of the length in respect of time of an expiration phase.

13. A control apparatus according to at least one of claims 1 to 12 **characterised in that** there is provided an inspiration time duration detection device for the generation of a signal indicative in respect of the length in respect of time of an inspiration phase.

14. A control apparatus according to at least one of claims 1 to 13 **characterised in that** there is provided a volume detection device for the detection of a breath volume.

15. A control apparatus according to at least one of claims 1 to 14 **characterised in that** there is provided a correlation detection device for the detection of a reverse correlation relationship of the instantaneous breath with a breath pattern which is representative in respect of previous breaths.

16. A control apparatus according to at least one of claims 1 to 15 **characterised in that** there is provided a curvature-ascertaining device for ascertaining the curvature of the respiratory gas flow pattern.

17. A control apparatus according to at least one of claims 1 to 16 **characterised in that** the apnoea detection device generates the switching signal on the basis of the signal produced on the part of the expiration time duration detection device.

18. A control apparatus according to at least one of claims 1 to 17 **characterised in that** the hypopnoea detection device generates the evaluation signal on the basis of the breath volume determined by the volume detection device and the reverse correlation relationship determined by the correlation detection device.

19. A control apparatus according to at least one of claims 1 to 18 **characterised in that** the normal respiration detection device generates the evaluation signal on the basis of the assessment result of the reverse correlation relationship.

20. A control apparatus according to at least one of claims 1 to 19 **characterised in that** the respiration stoppage detection device generates the evaluation signal on the basis of the respiratory gas flow signal.

21. A control apparatus according to at least one of claims 1 to 20 **characterised in that** there is provided a snore detection device for generating a signal classifying a snore event.

22. A control apparatus according to at least one of claims 1 to 21 **characterised in that** the snore detection device classifies the snore event on the basis of a zero crossing consideration of the first derivation of the respiratory gas pressure.

23. A control apparatus according to at least one of claims 1 to 22 **characterised in that** execution of switching over between the at least two pressure control modes is effected on the basis of assessment of a plurality of evaluation signals.

24. A control apparatus according to at least one of claims 1 to 23 **characterised in that** the evaluation signals are considered weighted with weighting factors.

25. A control apparatus according to at least one of claims 1 to 24 **characterised in that** the weighting factors are established on the basis of evaluations which are extracted from the signal indicative in respect of the respiratory gas flow.

26. A control apparatus according to claim 24 or claim 25 **characterised in that** the weighting factors are established in mutually matching relationship.

27. A control apparatus according to at least one of claims 24 to 26 **characterised in that** the variation in respect of time of the weighting factors is damped by a damping system.

28. A control apparatus according to at least one of claims 24 to 27 **characterised in that** the first pressure-determination mode responds to a hypopnoea event at a threshold value which is higher than the corresponding threshold value in the second pressure-determination mode.

29. A control apparatus according to at least one of claims 1 to 28 **characterised in that** in the second pressure-determination mode the respiratory gas pressure is adapted with a higher level of regulating dynamics.
